(19) Europäisches Patentamt
European Patent Office
Office européen des brevets

(11) Veröffentlichungsnummer: **0 363 726 B1**

(12) **EUROPÄISCHE PATENTSCHRIFT**

(45) Veröffentlichungstag der Patentschrift: **24.11.93**

(51) Int. Cl.[5]: **C07C 205/31**, C07C 201/12, A01N 33/20, A01N 35/04, A01N 43/78, C07C 205/34, C07C 205/44, C07D 277/24

(21) Anmeldenummer: **89117811.3**

(22) Anmeldetag: **27.09.89**

# (54) 1-Trifluormethyl-l-nitro-2-alkoxy-2-aryl-ethane, ihre Herstellung und sie enthaltende antimykotische Mittel.

(30) Priorität: **08.10.88 DE 3834326**

(43) Veröffentlichungstag der Anmeldung:
**18.04.90 Patentblatt 90/16**

(45) Bekanntmachung des Hinweises auf die Patenterteilung:
**24.11.93 Patentblatt 93/47**

(84) Benannte Vertragsstaaten:
**CH DE FR GB LI**

(56) Entgegenhaltungen:

**CHEMICAL ABSTRACTS, Band 62, 1965, Zusammenfassung 430d, Columbus, Ohio, US; I.L. KNUNYANTS et al.: "Aliphatic fluoro nitro compounds. III. Fluorine-containing nitro alcohols and ethers"**

(73) Patentinhaber: **BAYER AG**

**D-51368 Leverkusen(DE)**

(72) Erfinder: **Baasner, Bernd, Dr.**
**Hamberger Strasse 27 d**
**D-5090 Leverkusen 3(DE)**
Erfinder: **Beck, Günther, Dr.**
**Am Mittelberg 19**
**D-5090 Leverkusen 1(DE)**
Erfinder: **Heitzer, Helmut, Dr.**
**Höhenstrasse 84**
**D-5090 Leverkusen 3(DE)**
Erfinder: **Schaller, Klaus, Dr.**
**Am Sonnenschein 38**
**D-5600 Wuppertal 1(DE)**

**EP 0 363 726 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 1-Trifluormethyl-1-nitro-2-alkoxy-2-aryl-ethane der Formel (I)

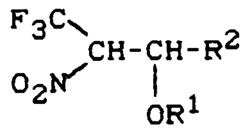 (I),

in der

$R^1$ für einen geradkettigen oder verzweigten Alkylrest und

$R^2$ für einen gegebenenfalls substituierten Aryl- oder Hetarylrest

stehen.

Vorzugsweise steht $R^1$ für einen geradkettigen oder verzweigten Alkylrest mit 1 bis 6 C-Atomen. Besonders bevorzugt sind Methyl, Ethyl, i-Propyl, n-Butyl, i-Butyl und t-Butyl.

Wenn $R^2$ Heteroatome enthält, kann es sich beispielsweise um 1 bis 2 Stickstoff-, Sauerstoff- und/oder Schwefelatome handeln. Wenn $R^2$ Substituenten enthält, kann es sich beispielsweise um 1 bis 3 Halogenatome, insbesondere Fluor- und/oder Chloratome, 1 bis 2 Nitrogruppen, 1 bis 2 Alkoxygruppen, insbesondere $C_1$- bis $C_6$-Alkoxygruppen, 1 bis 2 CHO-Gruppen oder um einen Rest des Typs

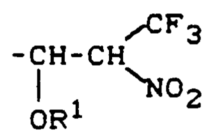

handeln, bei dem

$R^1$ die oben angegebene Bedeutung hat.

Vorzugsweise handelt es sich bei $R^2$ um einen 5- oder 6-gliedrigen, carbocyclischen oder heterocyclischen Arylrest, insbesondere um einen Phenyl- oder Thiazolrest.

Vorzugsweise enthält der Arylrest $R^2$ Substituenten. Bevorzugt handelt es sich bei den Substituenten um ein Fluoratom, 1 bis 3 Chloratome, eine Nitrogruppe, eine Methoxygruppe, eine CHO-Gruppe oder einen Rest des Typs

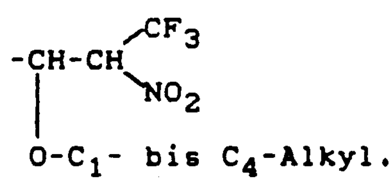

Besonders bevorzugte Reste $R^2$ sind: Dichlorthiazolyl, 3- und 4-Nitrophenyl, 4-Methoxyphenyl, 4-Formylphenyl, 2-, 3- und 4-Chlorphenyl, 3,4- und 2,5-Dichlorphenyl, 2,4,5-Trichlorphenyl, 2-Fluorphenyl und Reste des Typs

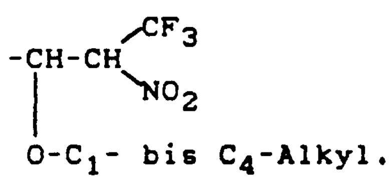

Die Verbindungen der Formel (I) weisen zwei asymmetrische C-Atome auf. Sie können deshalb in der R,R-, R,S-, S,R- und S,S-Form und in beliebigen Mischungen von zwei, drei oder allen diesen Formen

vorkommen.

Die vorliegende Erfindung betrifft die Verbindungen der Formel (I) jeweils sowohl in den einzelnen Formen (R,R-, R,S-, S,R- oder S,S-Form), als auch in beliebigen Mischungen von zwei, drei oder allen diesen Formen.

In solchen Mischungen kann beispielsweise eine beliebige erste Komponente (R,R-, R,S-, S,R- oder S,S-Form) in einem Anteil von 1 bis 70 Gew.-% und ergänzend zu 100 % eine zweite oder eine zweite und dritte oder eine zweite, dritte und vierte Komponente vorliegen. Vorzugsweise enthalten solche Mischungen alle vier Komponenten, d.h. die R,R-, R,S-, S,R- und S,S-Form einer Verbindung der Formel (I), wobei jede dieser Komponenten vorzugsweise in einer Menge zwischen 15 und 35 Gew.-% vorliegt und die Summe aller Komponenten 100 Gew.-% ergibt.

Die Zusammensetzung eines vorliegenden Gemisches optischer Isomerer einer Verbindung der Formel (I) kann auf an sich bekannte Weise verändert werden. Beispielsweise kann man eine Deprotonierung durchführen, gefolgt von einer Protonierung, vorzugsweise bei Temperaturen im Bereich -90 bis -100 °C mit Eisessig. Man kann auch chromatographische Methoden anwenden. Reine optische Isomere von Verbindungen der Formel (I) können aus Gemischen optischer Isomerer ebenfalls auf an sich bekannte Weise erhalten werden, z.B. durch Flüssigkeitschromatographie (LC).

Die vorliegende Erfindung betrifft weiterhin ein Verfahren zur Herstellung von 1-Trifluormethyl-1-nitro-2-alkoxy-2-aryl-ethanen der Formel (I)

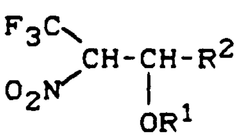

(I),

in der

$R^1$ für einen geradkettigen oder verzweigten Alkylrest und

$R^2$ für einen gegebenenfalls Heteroatome enthaltenden und gegebenenfalls substituierten Arylrest

stehen, das dadurch gekennzeichnet ist, daß man einen Aldehyd der Formel (II)

$R^2$-CHO (II),

in der

$R^2$ die bei Formel (I) angegebene Bedeutung hat,

mit einem Alkohol der Formel (III)

$R^1$-OH (III),

in der

$R^1$ die bei Formel (I) angegebene Bedeutung hat,

und Trifluormethylnitromethan in Gegenwart einer basische Gruppen enthaltenden Verbindung umsetzt.

Vorzugsweise und besonders bevorzugt werden solche Aldehyde der Formel (II) und solche Alkohole der Formel (III) eingesetzt, bei denen $R^2$ bzw. $R^1$ die weiter oben als bevorzugt und besonders bevorzugt bezeichneten Bedeutungen haben. Bei den basische Gruppen enthaltenden Verbindungen kann es sich beispielsweise um Ammoniak, primäre, sekundäre oder tertiäre Amine, Ammoniumacetat oder Aminosäuren handeln. Als Beispiele für Amine seien Pyridin und Piperidin genannt. Vorzugsweise wird 3-Aminopropionsäure (= $\beta$-Alanin) eingesetzt.

Die Einsatzmaterialien können beispielsweise in solchen Mengen eingesetzt werden, daß man auf 1 Äquivalent Aldehydgruppen, die in der Verbindung der Formel (II) enthalten sind, 0,8 bis 1,5 Mol eines Alkohols der Formel (III), mindestens 1 Mol Trifluormethylnitromethan und 0,1 bis 2 Mol der basische Gruppen enthaltenden Verbindung verwendet. Vorzugsweise setzt man auf 1 Äquivalent Aldehydgruppen, die in der Verbindung der Formel (II) enthalten sind, 1 bis 1,2 Mol eines Alkohols der Formel (III), 1 bis 1,8 Mol Trifluormethylnitromethan und 0,8 bis 1,2 Mol der basische Gruppen enthaltenden Verbindung ein.

Die erfindungsgemäße Umsetzung wird im allgemeinen in Gegenwart eines organischen Lösungsmittels durchgeführt. Bei solchen Lösungsmitteln kann es sich um inerte Lösungsmittel handeln, z.B. um Benzol oder Toluol, man kann aber auch einen Überschuß über die für die Reaktion erforderliche Menge des Alkohols der Formel (III) verwenden.

Da Fremdlösungsmittel in einem besonderen Schritt aus dem Reaktionsgemisch wieder abgetrennt werden müssen und Alkohole der Formel (III) im allgemeinen gut und kostengünstig zugänglich sind, arbeitet man vorzugsweise ohne Fremdlösungsmittel und mit überschüssigem Alkohol.

Das erfindungsgemäße Verfahren kann bei unterschiedlichen Temperaturen durchgeführt werden, beispielsweise bei solchen im Bereich -50 bis +100 °C. Bevorzugt sind Temperaturen im Bereich 0 bis 50 °C, besonders bevorzugt solche im Bereich 15 bis 30 °C. Bei höheren Temperaturen, beispielsweise über 30 °C und insbesondere über 50 °C, können zwar kürzere Reaktionszeiten angewendet werden, jedoch muß man dann im allgemeinen Verschlechterungen hinsichtlich der Selektivität in Kauf nehmen.

Geeignete Reaktionszeiten sind z.B. solche zwischen einer Stunde und einer Woche. Beim Arbeiten bei Temperaturen im Bereich 0 bis 30 °C werden im allgemeinen mit Reaktionszeiten von 10 bis 50 Stunden gute Ergebnisse erhalten. Optimale Reaktionszeiten kann man ermitteln, indem man den Reaktionsverlauf gaschromatographisch verfolgt und beispielsweise dann die Reaktion abbricht, wenn im Reaktionsgemisch kein Aldehyd der Formel (II) mehr vorhanden ist oder wenn ein besonders günstiges Verhältnis von gebildetem 1-Trifluormethyl-1-nitro-2-alkoxy-2-aryl-ethan der Formel (I) zu Nebenprodukten und gegebenenfalls noch vorhandenen Ausgangsstoffen vorliegt.

Die Aufarbeitung des Reaktionsgemisches kann auf verschiedene Weise erfolgen. Beispielsweise kann man zunächst die basische Gruppen enthaltende Verbindung oder Umwandlungsprodukte davon abfiltrieren, das Filtrat einengen und den verbleibenden Rückstand durch Umkristallisation, Destillation und/oder chromatographische Methoden weiter reinigen. Beispielsweise kann man das Reaktionsgemisch auch in Wasser einrühren. Wenn das gewünschte Reaktionsprodukt dann als Feststoff vorliegt, kann man diesen abfiltrieren durch Umkristallisation, Destillation und/oder chromatographische Methoden weiter reinigen. Wenn das gewünschte Reaktionsprodukt nach dem Einrühren in Wasser als Öl vorliegt, kann man dieses mit einem Lösungsmittel, z.B. Methylenchlorid, aufnehmen und beispielsweise auf chromatographischem Wege reinigen.

Die erfindungsgemäßen 1-Trifluormethyl-1-nitro-2-alkoxy-2-aryl-ethane der Formel (I) zeigen überraschenderweise eine antimykotische Wirkung, z.B. gegen Dermatophyten, Hefen und Schimmelpilze. Die Vorliegende Erfindung betrifft deshalb auch antimykotische Mittel, die dadurch gekennzeichnet sind, daß sie 1-Trifluormethyl-1-nitro-2-alkoxy-2-aryl-ethane der Formel (I) und gegebenenfalls übliche Zusatzstoffe enthalten.

## Beispiele

### Beispiel 1

60,0 g (0,427 Mol) 4-Chlorbenzaldehyd, enthaltend 0,427 Äquivalente Aldehydgruppen, wurden in 400 ml Methanol gelöst und zu der Lösung zunächst 67 g (0,52 Mol) Trifluormethylnitromethan und anschließend 38,0 g (0,427 Mol) β-Alanin hinzugefügt. Danach wurde das Reaktionsgemisch 24 Stunden lang bei 22 °C gerührt. Zur Aufarbeitung wurden die festen Bestandteile durch Filtration abgetrennt, mit Methanol gewaschen, das Filtrat und die Waschflüssigkeit vereinigt und im Vakuum eingeengt. Es hinterblieb ein öliges Gemisch mit kristallinen Anteilen. Die kristallinen Anteile wurden durch erneute Filtration abgetrennt, mit Methanol gewaschen und das Filtrat und die Waschflüssigkeit im Vakuum wieder eingeengt. Es wurden 88,1 g Rohprodukt erhalten, das gemäß gaschromatographischer Analyse folgende Zusammensetzung aufwies:
40,5 Gew.-% 4-Chlorbenzaldehyd,
13,4 Gew.-% 4-Chlorbenzaldehyd-dimethylacetal und
43,2 Gew.-% 1-Trifluormethyl-1-nitro-2-methoxy-2-p-chlorphenyl-ethan (Diastereomerengemisch 12,1 + 31,1 Gew.-%).

Dieses Rohprodukt wurde an 800 g Kieselgel mit Petrolether/Methylenchlorid (1:1) als Laufmittel chromatographisch aufgetrennt. Als erste Fraktion wurden 36,8 g 1-Trifluormethyl-1-nitro-2-methoxy-2-p-chlorphenyl-ethan (Diastereomerengemisch) als gelbes Öl mit einem Brechungsindex von $n_D^{20}$ : 1,4770 erhalten. Gemäß gaschromatographischer Bestimmung hatte dieses Produkt eine Reinheit von 96,9 %. Bezogen auf umgesetzten 4-Chlor-benzaldehyd betrug die Ausbeute also 60,4 % der Theorie.

Bei einer Wiederholung dieses Ansatzes, bei dem jedoch 96 Stunden lang bei 22 °C gerührt wurde, wurden nach der chromatographischen Aufarbeitung ein identisches Produkt in einer Ausbeute von 88,5 %, bezogen auf umgesetzten 4-Chlor-benzaldehyd, erhalten.

Beispiele 2 bis 18

Es wurde verfahren wie in Beispiel 1, mit einer Reaktionszeit von 24 Stunden, jedoch wurden die eingesetzten Alkohole und Aldehyde variiert. In einzelnen Fällen wurde auch ein anderes Laufmittel verwendet. Die Einzelheiten der durchgeführten Beispiele sind aus Tabelle 1 ersichtlich. In Tabelle 1 steht in der Spalte "$R^2$"

a für 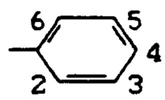 und

b für 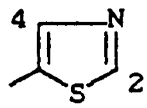 ,

in der Spalte "Laufmittel" steht A für Methylenchlorid/Petrolether 1:1, B für Methylenchlorid/Petrolether 3:1, C für Toluol/Essigsäureethylester 1:1 und D für Toluol und in der Spalte "Charakterisierung des Reaktionsproduktes" steht IR für charakteristische IR-Banden in $cm^{-1}$.

<u>Tabelle 1</u>

| Beispiel Nr. | Ausgangsprodukte der Formeln (II) und (III) und Reaktionsprodukt der Formel (I) mit $R^1$ | | $R^2$ | Laufmittel | Charakterisierung des Reaktionsproduktes | Ausbeute an gereinigtem Produkt (% d.Th.), bezogen auf umgesetztes Ausgangsprodukt der Formel (II) |
|---|---|---|---|---|---|---|
| 2 | $CH_3$ | a | 4-$NO_2$ | A | Schmelzpunkt: 105°C | 77 |
| 3 | $CH_3$ | a | 3-$NO_2$ | A | IR: 1591, 1530, 1360, 1265, 1182, 1148, 1112, 884, 781, 740, 698, 682 | 63 |
| 4 | $CH_3$ | a | 4-CHO | B | IR: 1702, 1580, 1372, 1335, 1265, 1210, 1184, 1145, 1109, 1073, 888, 832, 772, 721 | 47 |
| 5 | $CH_3$ | a | 3,4-Di-Cl | A | IR: 1580, 1472, 1374, 1325, 1259, 1213, 1179, 1142, 1108, 785 | 81 |
| 6 | $CH_3$ | a | 4-Cl | A | IR: 1573, 1495, 1362, 1325, 1259, 1217, 1189, 1143, 1109, 887, 832, 780 | 60,4 |
| 7 | $CH_3$ | a | 2,4-Di-Cl | A | IR: 1579, 1477, 1366, 1324, 1260, 1211, 1186, 1140, 1108, 797 | 79 |
| 8 | $CH_3$ | a | 3-Cl | A | IR: 1576, 1370, 1326, 1259, 1216, 1189, 1145, 1110, 796, 780, 710, 692 | 82 |
| 9 | $CH_3$ | a | 2-Cl | A | IR: 1578, 1370, 1329, 1261, 1213, 1188, 1146, 1107, 783, 760 | 67 |

Tabelle 1 (Fortsetzung)

| Beispiel Nr. | Ausgangsprodukte der Formeln (II) und (III) und Reaktionsprodukt der Formel (I) mit $R^1$ | | $R^2$ | Laufmittel | Charakterisierung des Reaktionsproduktes | Ausbeute an gereinigtem Produkt (% d.Th.), bezogen auf umgesetztes Ausgangsprodukt der Formel (II) |
|---|---|---|---|---|---|---|
| 10 | $CH_3$ | a | 2,6-Di-Cl | A | Siedepunkt: 145°C bei 22 mbar (nach der Chromatographie) | 58 |
| 11 | n-Butyl | a | $4-NO_2$ | | Schmelzpunkt: 72°C*) | 61 |
| 12 | $CH(CH_3)_2$ | a | $4-NO_2$ | | Schmelzpunkt: 94°C*) | 58 |
| 13 | n-Butyl | b | 2,4-Di-Cl | D | IR: 1580, 1515, 1422, 1366, 1321, 1257, 1197, 1093, 1064, 896 | 67 |
| 14 | $CH(CH_3)_2$ | b | 2,4-Di-Cl | B | Schmelzpunkt: 75°C | 48 |
| 15 | $CH_3$ | a | 2-F | A | IR: 1578, 1495, 1462, 1369, 1330, 1263, 1189, 1145, 1107, 887, 764 | 73 |
| 16 | $CH_3$ | a | 4-CHO | B | Schmelzpunkt: 170-172°C | 53 |
| 17 | $CH_3$ | a | 3-CHO | A | IR: 1580, 1372, 1355, 1326, 1266, 1211, 1190, 1153, 1097, 892, 851, 769, 719 | 66 |
| 18 | n-Butyl | a | 4-CHO | | Schmelzpunkt: 157°C*) | 71 |

*) Diese Produkte wurden nicht chromatographiert, sondern aus Petrolether umkristallisiert.

Beispiel 19 (Test der antimykotischen Wirksamkeit)

Die in vitro-Prüfungen wurden in Reihenverdünnungstests durchgeführt, wobei der Einfluß der geprüften Substanzen auf Keimeinsaaten in verschiedenen Medien untersucht und jeweils die minimale Hemmkonzentration der geprüften Substanzen ermittelt wurde.

Als Keime wurden verwendet:

Als Beispiel für Dermatophyten:

A Trichophyton mentagrophytes.

Als Medium hierfür wurde sog. Kimmig-Bouillon (5 g Glycerin, 13 g Nutrient Broth, 8,6 g Bacto Repton, 9,0 g NaCl und 10 g Glukose vermischt mit 1 l entmineralisiertem Wasser) verwendet. In dieses Medium wurden 1 x $10^5$ Keime A pro ml eingesetzt und 5 Tage lang bei 28 °C bebrütet.

Als Beispiel für Schimmelpilze:

B Aspergillus fumigatus.

Es wurde das gleiche Medium, die gleiche Menge Keime und die gleiche Bebrütungstemperatur wie bei A angewendet. Die Bebrütungszeit betrug 4 Tage.

Als Beispiele für Hefen:

C Candida albicans.

Als Medium hierfür wurde Yeast Nitrogen Base (Difco) verwendet. In dieses Medium wurden 1 x $10^4$ Keime C pro ml eingesetzt und 3 Tage lang bei 37 °C bebrütet.

D Torulopsis glabrata.

Es wurde das gleiche Medium, die gleiche Menge Keime, die gleiche Bebrütungsdauer und die gleiche Bebrütungstemperatur wie bei C angewendet.

Die Ergebnisse sind in Tabelle 2 zusammengestellt.

Tabelle 2

| geprüfte Substanz | Minimale Hemmkonzentration (µg/ml) für Keime von | | | |
|---|---|---|---|---|
| | A | B | C | D |
| aus Beispiel 5 | 2 | 8 | <1 | <1 |
| aus Beispiel 6 | 1 | 16 | 2 | <1 |
| aus Beispiel 7 | 4 | 64 | 4 | <1 |
| aus Beispiel 8 | 2 | 8 | 2 | <1 |

## Patentansprüche

**1.** 1-Trifluormethyl-1-nitro-2-alkoxy-2-aryl-ethane der Formel (I)

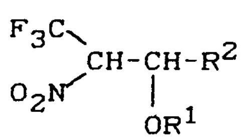

(I),

in der

$R^1$ für einen geradkettigen oder verzweigten Alkylrest und

$R^2$ für einen gegebenenfalls substituierten Aryl- oder Hetarylrest

stehen,

**2.** 1-Trifluormethyl-1-nitro-2-alkoxy-2-aryl-ethane gemaß Anspruch 1, dadurch gekennzeichnet, daß in Formel (I) $R^1$ für einen geradkettigen oder verzweigten Alkylrest mit 1 bis 6 C-Atomen steht und $R^2$ gegebenenfalls als Heteroatome 1 bis 2 Stickstoff-, Sauerstoff- und/oder Schwefelatome und gegebenenfalls als Substituenten 1 bis 3 Halogenatome, 1 bis 2 Nitrogruppen, 1 bis 2 Alkoxygruppen, 1 bis 2 CHO-Gruppen oder einen Rest des Typs

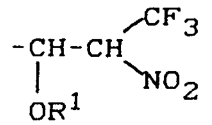,

bei dem
$R^1$ die bei Formel (I) angegebene Bedeutung hat,
enthält.

3. 1-Trifluormethyl-1-nitro-2-alkoxy-2-aryl-ethane gemaß Ansprüchen 1 und 2, dadurch gekennzeichnet, daß es sich bei $R^2$ um einen 5- oder 6-gliedrigen carbocyclischen oder heterocyclischen Ring handelt.

4. Verfahren zur Herstellung von 1-Trifluormethyl-1-nitro-2-alkoxy-2-aryl-ethanen der Formel (I)

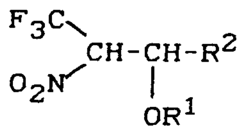 (I),

in der
$R^1$ für einen geradkettigen oder Verzweigten Alkylrest und
$R^2$ für einen gegebenenfalls Heteroatome enthaltenden und gegebenenfalls substituierten Arylrest
stehen, dadurch gekennzeichnet, daß man einen Aldehyd der Formel (II)

$R^2$-CHO (II),

in der
$R^2$ die bei Formel (I) angegebene Bedeutung hat,
mit einem Alkohol der Formel
(III)

$R^1$-OH (III),

in der
$R^1$ die bei Formel (I) angegebene Bedeutung hat,
und Trifluormethylnitromethan in Gegenwart einer basische Gruppen enthaltenden Verbindung umsetzt.

5. Verfahren nach Anspruch 4, dadurch gekennzeichnet, daß man bezogen auf 1 Äquivalent Aldehydgruppen, die in der Verbindung der Formel (II) enthalten sind, 0,8 bis 1,5 Mol eines Alkohols der Formel (III), mindestens 1 Mol Trifluormethylnitromethan und 0,1 bis 2 Mol einer basische Gruppen enthaltenden Verbindung einsetzt.

6. Verfahren nach Ansprüchen 4 und 5, dadurch gekennzeichnet, daß man es bei -50 bis +100 °C durchführt.

7. Antimykotisches Mittel, dadurch gekennzeichnet, daß es 1-Trifluormethyl-1-nitro-2-alkoxy-2-aryl-ethane der Formel (I)

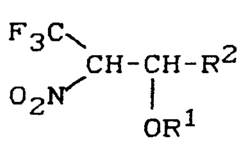 (I),

in der
$R^1$ für einen geradkettigen oder verzweigten Alkylrest und
$R^2$ für einen gegebenenfalls substituierten Aryl- oder Hetarylrest
stehen,
und gegebenenfalls übliche Zusatzstoffe enthalt.

**Claims**

**1.** 1-Trifluoromethyl-1-nitro-2-alkoxy-2-aryl-ethanes of the formula (I)

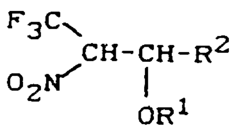

(I)

in which
$R^1$ represents a straight-chain or branched alkyl radical and
$R^2$ represents an optionally substituted aryl or hetaryl radical.

**2.** 1-Trifluoromethyl-1-nitro-2-alkoxy-2-aryl-ethanes according to Claim 1, characterized in that in formula (I) $R^1$ represents a straight-chain or branched alkyl radical having 1 to 6 C atoms and $R^2$ optionally contains 1 or 2 nitrogen, oxygen and/or sulphur atoms as heteroatoms and optionally contains 1 to 3 halogen atoms, 1 or 2 nitro groups, 1 or 2 alkoxy groups, 1 or 2 CHO groups or a radical of the type

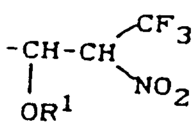

in which
$R^1$ has the meaning indicated in formula (I),
as substituents.

**3.** 1-Trifluoromethyl-1-nitro-2-alkoxy-2-aryl-ethanes according to Claims 1 and 2, characterized in that $R^2$ is a 5- or 6-membered carbocyclic or heterocyclic ring.

**4.** Process for the preparation of 1-trifluoromethyl-1-nitro-2-alkoxy-2-aryl-ethanes of the formula (I)

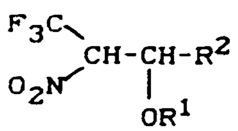

(I)

in which
$R^1$ represents a straight-chain or branched alkyl radical and
$R^2$ represents an optionally substituted aryl radical optionally containing heteroatoms,
characterized in that an aldehyde of the formula (II)

$R^2$-CHO (II)

in which
$R^2$ has the meaning indicated in formula (I),
is reacted with an alcohol of the formula (III)

$R^1$-OH (III)

in which

$R^1$ has the meaning indicated in formula (I),

and trifluoromethylnitromethane in the presence of a compound containing basic groups.

5. Process according to Claim 4, characterized in that, based on 1 equivalent of aldehyde groups which are contained in the compound of the formula (II), 0.8 to 1.5 moles of an alcohol of the formula (III), at least 1 mole of trifluoromethylnitromethane and 0.1 to 2 moles of a compound containing basic groups are employed.

6. Process according to Claims 4 and 5, characterized in that it is carried out at -50 to +100 °C.

7. Antimycotic agent, characterized in that it contains 1-trifluoromethyl-1-nitro-2-alkoxy-2-aryl-ethanes of the formula (I)

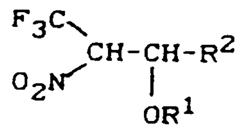

(I)

in which

$R^1$ represents a straight-chain or branched alkyl radical and

$R^2$ represents an optionally substituted aryl or hetaryl radical,

and, if appropriate, customary additives.

## Revendications

1. 1-trifluorométhyl-1-nitro-2-alkoxy-2-aryl-éthanes de formule (I)

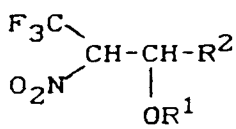

(I),

dans laquelle

$R^1$ est un reste alkyle à chaîne droite ou ramifiée et

$R^2$ est un reste aryle ou hétaryle éventuellement substitué.

2. 1-trifluorométhyl-1-nitro-2-alkoxy-2-aryl-éthanes suivant la revendication 1, caractérisés en ce que dans la formule (I), $R^1$ représente un reste alkyle à chaîne droite ou ramifiée ayant 1 à 6 atomes de carbone et $R^2$ contient, le cas échéant, comme hétéro-atomes 1 ou 2 atomes d'azote, d'oxygène et/ou de soufre et, le cas échéant, comme substituants, 1 à 3 atomes d'halogènes, 1 à 2 groupes nitro, 1 à 2 groupes alkoxy, 1 à 2 groupes CHO ou un reste du type

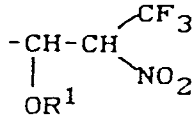

,

dans lequel

$R^1$ a la définition indiquée pour la formule (I).

3. 1-trifluorométhyl-1-nitro-2-alkoxy-2-aryl-éthanes suivant les revendications 1 et 2, caractérisés en ce que $R^2$ représente un noyau carbocyclique ou hétérocyclique pentagonal ou hexagonal.

4. Procédé de production de 1-trifluorométhyl-1-nitro-2-alkoxy-2-aryl-éthanes de formule (I)

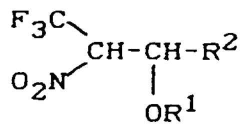

(I),

dans laquelle
$R^1$ représente un reste alkyle à chaîne droite ou ramifiée et
$R^2$ représente un reste aryle contenant éventuellement des hétéro-atomes et éventuellement substitué,
caractérisé en ce qu'on fait réagir un aldéhyde de formule (II)

$R^2$-CHO (II),

dans laquelle
$R^2$ a la définition indiquée pour la formule (I),
avec un alcool de formule (III)

$R^1$-OH (III),

dans laquelle
$R^1$ a la définition indiquée pour la formule (I)
et le trifluorométhylnitrométhane en présence d'un composé contenant des groupes basiques.

5. Procédé suivant la revendication 4, caractérisé en ce qu'on utilise, pour un équivalent de groupes aldéhyde qui sont contenus dans le composé de formule (II), 0,8 à 1,5 mole d'un alcool de formule (III), au moins 1 mole de trifluorométhylnitrométhane et 0,1 à 2 moles d'un composé contenant des groupes basiques.

6. Procédé suivant les revendications 4 et 5, caractérisé en ce qu'il est mis en oeuvre entre -50 et +100 °C.

7. Composition antimycosique, caractérisée en ce qu'elle contient des 1-trifluorométhyl-1-nitro-2-alkoxy-2-aryl-éthanes de formule (I)

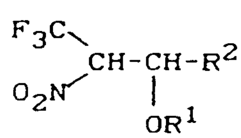

(I),

dans laquelle
$R^1$ est un reste alkyle à chaîne droite ou ramifiée et
$R^2$ est un reste aryle ou hétaryle éventuellement substitué,
et, le cas échéant, des additifs classiques.